# EUROPÄISCHE PATENTSCHRIFT

(11) **EP 2 599 511 B1**
(45) Veröffentlichungstag und Bekanntmachung des Hinweises auf die Patenterteilung: **13.08.2014**
(21) Anmeldenummer: 11181527.0
(22) Anmeldetag: 30.11.2011
(51) Int. Cl.: A61M 5/145

(54) **Antriebsvorrichtung zum linearen Bewegen eines Infusionsspritzenkolbens, Infusionspumpe und Verfahren zum Auswechseln einer Infusionsspritze**
Drive device for linear movement of an infusion syringe plunger, infusion pump and method for replacement of an infusion syringe
Dispositif d'actionnement pour le déplacement linéaire d'un piston pour seringues à perfusion, pompe à perfusion et procédé de changement d'une seringue à perfusion

(43) Veröffentlichungstag der Anmeldung: 05.06.2013
(73) Patentinhaber: B. Braun Melsungen AG, 34212 Melsungen (DE)
(72) Erfinder: Heitmeier, Rolf, 34225 Baunatal (DE); Wildner, Rene, 34212 Melsungen (DE); Gerlach, Hans-Josef, 34431 Marsberg (DE)
(74) Vertreter: Winter, Brandl, Fürniss, Hübner, Röss, Kaiser, Polte - Partnerschaft

(56) Entgegenhaltungen:
- EP-A1- 0 514 907
- EP-A2- 1 110 569
- WO-A1-2011/039250
- US-A- 5 879 360

## Beschreibung

Die Erfindung betrifft eine Antriebsvorrichtung zum linearen Bewegen eines Infusionsspritzenkolbens einer an einer Infusionspumpe angeordneten Infusionsspritze mit einer Antriebskopfeinheit umfassend eine Hauptanlageeinrichtung zum Abstützen des Infusionsspritzenkolbens, eine Fixiereinrichtung zum Festlegen des Infusionsspritzenkolbens an der Hauptanlageeinrichtung sowie Mittel zum Auslösen der Fixiereinrichtung und mit einer Vortriebseinrichtung für die Antriebskopfeinheit umfassend eine motorisch antreibbare Vortriebsspindel und eine wenigstens eine radial bewegbare Mutterschale aufweisende mehrteilige Vortriebsspindelmutter, wobei die Antriebskopfeinheit zusätzlich eine Detektiereinrichtung zum Detektieren des Infusionsspritzenkolbens vor der Hauptanlageeinrichtung umfasst.

Darüber hinaus betrifft die Erfindung eine Infusionspumpe mit einer Antriebskopfeinheit zum Bewegen eines Infusionsspritzenkolbens einer an der Infusionspumpe angeordneten Infusionsspritze und mit einer Vortriebseinrichtung zum Treiben der Antriebkopfeinheit.

Des Weiteren betrifft die Erfindung ein Verfahren zur Inbetriebnahme einer Infusionsspritze an einer Infusionspumpe mit einer Antriebskopfeinheit zum linearen Bewegen eines Infusionsspritzenkolbens, bei welchem wenigstens eine radial bewegbare Mutterschale einer Vortriebsspindelmutter radial von einer Vortriebsspinde! einer Vortriebseinrichtung zum Treiben der Antriebskopfeinheit radial ausgerückt wird, sodass die Antriebskopfeinheit einer Kolbenplatte des Infusionsspritzenkolbens händisch schnell axial zugestellt werden kann, und bei welchem zum Verhindern einer unbeabsichtigten Bolusgabe die Kolbenplatte hierbei mit einem der Antriebskopfeinheit zugeordneten Kontaktelement detektiert wird, bevor die Kolbenplatte mit einer Hauptanlagefläche einer Hauptanlageeinrichtung der Antriebskopfeinheit in Kontakt kommt.

Insbesondere gattungsgemäße Antriebsvorrichtungen und diesbezügliche Spritzenpumpen sind aus dem Stand der Technik gut bekannt und werden seit langem erfolgreich in der Infusionstherapie eingesetzt.

Beispielsweise ist aus der WO 2011/039250 A1 eine Methode zum Verhindern einer unbeabsichtigten Bolusgabe hinsichtlich einer händischen Zustellbewegung einer Schiebeeinrichtung für einen Infusionsspritzenkolben insbesondere nach einem Wechsel einer Infusionsspritze an einer Infusionspumpe sowie die entsprechende Infusionspumpe hierfür bekannt. Die Infusionspumpe weist neben der Schiebeeinrichtung zum Schieben des Infusionsspritzenkolbens insbesondere eine Infusionsspritzenaufnahme zum Festlegen einer Infusionsspritze, einen eine zweiteilige Vortriebsspindelmutter umfassende Vortrieb zum Treiben der Schiebeeinrichtung sowie eine Einrichtung zum Blockieren der händischen Zustellbewegung der Schiebeeinrichtung auf. Hierbei zeichnet sich der Vortrieb durch eine Vortriebsspindel und eine Vortriebskupplung zum Kuppeln bzw. Entkuppeln der Schiebeeinrichtung mit bzw. von der Vortriebsspindel aus, wodurch die Schiebeeinrichtung im entkuppelten Zustand der Kupplung vorteilhafter Weise händisch schnell dem Infusionsspritzenkolben der neu eingelegten Infusionsspritze zugestellt werden kann. Dieses schnelle händische Zustellen ist besonders in kritischen Situationen vorteilhaft, wenn es geboten ist, einem Patienten schnell eine wichtige Infusion zuführen zu müssen. Um hierbei jedoch die Gefahr einer nicht ungefährlichen unbeabsichtigten Bolusgabe an dem Patienten reduzieren zu können, verfügt die Infusionspumpe über eine Blockiereinrichtung, mittels welcher die händische Zustellbewegung schnell unterbunden werden kann, wenn die Schiebeeinrichtung dem Infusionsspritzenkolben händisch ausreichend nah zugestellt wurde. Die Blockiereinrichtung zeichnet sich im Speziellen durch eine der Vortriebsspindel nebengeordneten Kontrollgewindewelie mit einer hiermit unlösbar korrespondierenden Gewindewellenmutter und durch eine Bremseinrichtung für die Kontrollgewindewelie aus. Die Gewindewellenmutter ist mit der Schiebeeinrichtung fest verbunden, sodass die händische Zustellbewegung sofort blockiert, sobald die Bremseinrichtung eine Rotation der Kontrollgewindewelie blockt. Die Blockiereinrichtung wird durch eine der Schiebeeinrichtung zugeordneten Signaleinrichtung gesteuert, welche zumindest einen Sensor an der Schiebeeinrichtung umfasst, der eine Annäherung der Schiebeeinrichtung an den Infusionsspritzenkolben detektieren kann, wobei die Bremseinrichtung die Kontrollgewindewelle blockt, wenn eine gewissen Annäherung des Infusionsspritzenkolbens an der Schiebeeinrichtung erreicht oder überschritten ist. Hierdurch kann ein unbeabsichtigtes bzw. kritisches Anstoßen an den Infusionsspritzenkolben im Zuge einer schnellen händischen Zustellbewegung betriebssicher verhindert werden. Zum Blockieren der Kontrollgewindewelle weist die Blockiereinrichtung eine elektromagnetische Bremse auf, welche mit der Signaleinrichtung elektrische verbunden ist.

Aus der US 5 879 360 A ist eine Spritzenpumpe mit einem Antriebskopf auf einer Führungsspindel bekannt, der bewirkt, dass der Kolben der Spritze vorgeschoben wird, um den Inhalt der Spritze kontrolliert abzugeben. Dazu weist der Antriebskopf ein Sensormodul auf, der den Kontakt zum Kolben darstellt. Der Antriebskopf trägt federbelastete Sperrklinken, die eingreifen, um eine unerwünschte Bewegung des Kolbens, zum Beispiel Absaugen, zu verhindern.

Die Druckschrift EP 0 514 907 A1 zeigt ein Spritzenkolben-Antriebssystem für Spritzen verschiedener Größen mit einem einzigen Kolbenmitnehmer. Die Druckfläche des Kolbenmitnehmers weist einen Kolbendetektor auf. Steht diese mit dem Kolbenkopf in Kontakt, gelangt der Verstellschraubenrnechanismus wieder in Eingriff mit dem Gewinde der Verstellschraubenspindel.

Es ist Aufgabe vorliegender Erfindung ein Auswechseln einer Infusionsspritze an einer Infusionspumpe einfacher und dennoch insbesondere hinsichtlich der Gefahr einer unbeabsichtigten Bolusgabe an einen Patienten sicherer zu gestalten.

Die Aufgabe der Erfindung wird zum einen von einer Antriebsvorrichtung zum linearen Bewegen eines Infusionsspritzenkolbens einer an einer Infusionspumpe angeordneten Infusionsspritze mit einer Antriebskopfeinheit umfassend eine Hauptanlageeinrichtung zum Abstützen des Infusionsspritzenkolbens, eine Fixiereinrichtung zum Festlegen des Infusionsspritzenkolbens an der Hauptanlageeinrichtung sowie Mittel zum Auslösen der Fixiereinrichtung und mit einer Vortriebseinrichtung für die Antriebskopfeinheit umfassend eine motorisch antreibbare Vortriebsspindel und eine wenigstens eine radial bewegbare Mutterschale aufweisende mehrteilige Vortriebsspindelmutter, wobei die Antriebskopfeinheit zusätzlich eine Detektiereinrichtung zum Detektieren des Infusionsspritzenkolbens vor der Hauptanlageeinrichtung umfasst, gelöst, und wobei die Detektiereinrichtung eine Mehr-Schwellenwert- Sensoreinheit mit einer mindestens zweistufigen Vorfelderfassungseinrichtung zum Erfassen unterschiedlicher Positionen des Infusionsspritzenkolbens, bevor eine Kolbenplatte des Infusionsspritzenkolbens mit der der Hauptanlageeinrichtung in Kontakt kommt, aufweist.

Der Begriff "Mehr-Schwellenwert-Sensoreinheit" beschreibt im Sinne der Erfindung eine Einrichtung zum Detektieren unterschiedlicher Positionen des vor der Antriebskopfeinheit platzierten Infusionsspritzenkolbens, insbesondere dessen Kolbenplatte, bevor sich der Infusionsspritzenkolben mittels der Kolbenplatte an der Hauptanlageeinrichtung der Antriebskopfeinheit abstützt.

Hierbei kann die Mehr-Schwellenwert-Sensoreinheit aus mehreren Einzelsensoren bestehen, die an der bzw. in der Antriebskopfeinheit angeordnet sind und die dort zu einer Sensoreinheit funktional untereinander verbunden sein können. Oder die Sensoreinheit besteht aus einem einzigen Bauteil, in dessen Gehäuse wenigstens zwei Sensoren zumindest teilweise umhaust sind, je nachdem welche bauliche Ausführung in einer konkreten Ausführung vorteilhafter verbaut werden kann.

Vorteilhafter Weise wird durch die Mehr-Schwellenwert-Sensoreinheit ein Ablauf zur ordnungsgemäßen Inbetriebnahme einer Infusionspumpe insbesondere nach einem Infusionsspritzenwechsel mindestens zweistufig automatisiert, bevor die Hauptanlageeinrichtung mit der Kolbenplatte des Infusionsspritzenkolbens in Kontakt treten kann, wodurch die Inbetriebnahme der Infusionspumpe wesentlich effektiver und zudem betriebssicherer vorgenommen werden kann.

Dies liegt zum einen daran, dass ein Blockieren einer händischen Zustellung der Antriebskopfeinheit mittels einer ersten Stufe der Vorfelderfassungseinrichtung ausgelöst werden kann, wobei eine weitere Zustellung dann unmittelbar mit einer motorgetriebenen Zustellung fortgesetzt werden kann, welche durch eine zweite Stufe der Vorfelderfassungseinrichtung idealerweise nahtlos in eine definierte motorgetriebene Referenzfahrt übergeht, bei welcher die Vortriebsspindel nur noch eine definierte Anzahl an Umdrehungen durchführt. Hierdurch kann die Antriebskopfeinheit einerseits außergewöhnlich schnell und andererseits außerordentlich exakt an der Kolbenplatte heran gefahren und einsatzbereit an der Kolbenplatte platziert werden.

Insofern wird die Aufgabe der Erfindung zum anderen auch von einem Verfahren zum Auswechseln einer Infusionsspritze an einer Infusionspumpe mit einer Antriebskopfeinheit zum linearen Bewegen eines Infusionsspritzenkolbens gelöst, bei welchem wenigstens eine radial bewegbare Mutterschale einer Vortriebsspindelmutter radial von einer Vortriebsspindel einer Vortriebseinrichtung zum Treiben der Antriebskopfeinheit radial ausgerückt wird, sodass die Antriebskopfeinheit einer Kolbenplatte des Infusionsspritzenkolbens händisch schnell axial zugestellt werden kann, und bei welchem zum Verhindern einer unbeabsichtigten Bolusgabe die Kolbenplatte hierbei mit einem der Antriebskopfeinheit zugeordneten Kontaktelement detektiert werden kann, bevor die Kolbenplatte mit einer Hauptanlagefläche einer Hauptanlageeinrichtung der Antriebskopfeinheit in Kontakt kommt, wobei
- in einem ersten Detektierschritt das Kontaktelement ein erstes Sensorelement auslöst, wodurch das händische Zustellen der Antriebskopfeinheit mittels der Vortriebsspindelmutter und der Vortriebsspindel blockiert wird, indem die wenigstens eine radial bewegbare Mutterschale radial an der Vortriebsspindel eingerückt wird, wobei anschließend mittels der Vortriebseinrichtung die Antriebskopfeinheit der Kolbenplatte motorgetrieben weiter zugestellt wird, dass
- in einem zweiten Detektierschritt das Kontaktelement ein zweites Sensorelement auslöst, wodurch eine motorgetriebene Referenzfahrt der Antriebskopfeinheit bis an die Hauptanlagefläche der Hauptanlageeinrichtung heran initiiert wird, und dass
- in einem dritten Detektierschritt im Zuge eines Kontakts der Kolbenplatte mit der Hauptanlagefläche die motorgetriebene Referenzfahrt gestoppt wird, und Haltebügel einer Fixiereinrichtung für die Kolbenplatte geschlossen werden, wodurch die Kolbenplatte an der Hauptanlageeinrichtung festgelegt wird.

Durch dieses Mehrfach-Detektieren kann die Betriebssicherheit an einer Infusionspumpe weiter erhöht werden, ohne dass es hierbei zu einer Verzögerung beim Auswechseln der Infusionsspritze kommt.

Vorteilhafter Weise wird die händische Zustellung der Antriebskopfeinheit blockiert, bevor das Kontaktelement das zweite Sensorelement auslöst. Hierdurch kann insbesondere sichergestellt werden, dass die Vortriebsspindelmutter korrekt an der Vortriebsspindel eingerastet ist, bevor die Referenzfahrt mittels des zweiten Sensors der Mehr-Schwellenwert-Sensoreinheit eingeleitet wird, sobald dieser zweite Sensor mittels des Kontaktelements entsprechend ausgelöst wird. Somit kann stets die exakte Position der Antriebskopfeinheit gegenüber der Kolbenplatte gewährleistet werden, bevor die Referenzfahrt beginnt.

Eine bevorzugte Verfahrensvariante sieht vor, dass die motorgetriebene Referenzfahrt anhand einer definierten Anzahl an Motorschritten durchgeführt wird. Durch die definierte Anzahl an Motorschritten kann eine genau vorherbestimmte Anzahl an Umdrehungen der Vortriebsspindel und damit auch ein definierter Zustellweg für die Antriebskopfeinheit insbesondere auf den letzten Millimetern realisiert werden, sodass die Antriebskopfeinheit vor der Kolbenplatte immer besonders exakt platziert werden kann.

Konstruktiv ist es des Weiteren besonders vorteilhaft, wenn die Mehr-Schwellenwert-Sensoreinheit wenigstens zwei mittels eines einzigen Geberelementes auslösbare Sensoren aufweist, wobei das Geberelement ein über die Hauptanlageeinrichtung hinausragendes Kontaktelement umfasst.

Da die Einbauräumlichkeiten an der Antriebskopfeinheit, insbesondere an der Hauptanlageeinrichtung für eine Kolbenplatte des Infusionsspritzenkolbens, meistens sehr knapp bemessen sind, ist es besonders vorteilhaft, wenn die wenigstens zwei Sensoren der Mehr-Schwellenwert-Sensoreinheit durch nur ein einziges Geberelement ausgelöst werden können.

Vorteilhafter Weise gestaltet das Kontaktelement sogleich das Geberelement aus, sodass die Antriebskopfeinheit noch kompakter gebaut werden kann.

Es versteht sich, dass die vorstehend erwähnten auslösbaren Sensoren von den unterschiedlichsten Sensoreinrichtungen realisiert sein können. Beispielsweise können Hallsensoren zum Einsatz kommen.

Eine konstruktiv besonders bevorzugte Ausführungsvariante sieht vor, dass die Detektiereinrichtung vorzugsweise wenigstens zwei Lichtschranken umfasst, die axial hintereinander innerhalb der Antriebskopfeinheit angeordnet sind. Insbesondere die beiden derart hintereinander angeordneten Lichtschranken können baulich besonders einfach mittels eines einzigen Geberelementes angesprochen werden.

Hierbei bedeutet "axial" im Wesentlichen fluchtend zu der Zustellbewegung der Antriebskopfeinheit ausgerichtet, sodass die Betätigungsachse des Geberelementes vorteilhafter Weise mit der Schiebeachse des Infusionsspritzenkolbens übereinstimmt oder parallel zu dieser angeordnet ist. Insofern kann das Geberelement vorteilhafter Weise problemlos bauteilidentisch mit dem Kontaktelement ausgebildet sein.

Eine weitere konstruktive Vereinfachung kann an der Antriebskopfeinheit erzielt werden, wenn wenigstens eine der beiden Lichtschranken als Gabellichtschranke ausgestaltet ist.

Darüber hinaus ist es vorteilhaft, wenn die Hauptanlageeinrichtung einen Membranteller aufweist, welcher ein ein Kontaktelement umfassendes Geberelement zum Schalten von Sensoren der Mehr-Schwellenwert-Sensoreinheit umfasst.

Beispielsweise verkörpert ein federvorgespannte Membranteller zumindest ein Bauteil bzw. eine Bauteilgruppe der Mittel zum Auslösen der Fixiereinrichtung für die Kolbenplatte. Ist dann in diesem federvorgespannten Membranteller sogleich das Mehr-Schwellenwert-Sensoreinheit-Geberelement angeordnet, kann die Detektiereinrichtung baulich weiter vereinfacht werden.

Vorzugsweise ist das einziges Geberelement der Mehr-Schwellenwert-Sensoreinheit innerhalb der Mittel zum Auslösen der Fixiereinrichtung angeordnet ist, sodass insbesondere die Hauptanlageeinheit noch kompakter gebaut werden kann.

Eine weitere sehr vorteilhafte Ausführungsvariante sieht vor, dass ein einziges Geberelement der Mehr-Schwellenwert-Sensoreinheit einen Vorlaufweg mit einem Wert zwischen 2 mm und 10 mm, vorzugsweise mit einem Wert von 4 mm, aufweist, bevor eine erste Stufe der zweistufigen Vorfelderfassungseinrichtung von dem einzigen Geberelement auslösbar ist. Durch einen derartig kurz gewählten Vorlaufweg ist die Gefahr verringert, dass bei einer zu heftigen händischen Zustellbewegung die Blockiereinrichtung nicht schnell genug reagieren kann und die Kolbenplatte kritisch an die Hauptanlageeinrichtung anschlägt.

Weist die Antriebsvorrichtung wenigstens eine in Abhängigkeit der Detektiereinrichtung arbeitende gemeinsame Regel- und/oder Steuereinrichtung zum automatischen radialen Einrücken der wenigstens einen radial bewegbaren Mutterschale an der Vortriebsspindel und zum Initiieren einer mittels der radial eingerückten Vortriebsspindelmutter durchführbaren Referenzfahrt der Hauptanlageeinrichtung bis an den Infusionskolbens heran auf, kann ein Zusammenspiel dieser Funktionen besonders vorteilhaft miteinander verknüpft werden.

Damit ein Abfallen des Energiezustands und damit auch der Magnetfelder an den beiden Elektrohaltemagneten besonders schnell erzielt werden kann, ist es vorteilhaft, wenn die Regel- und/oder Steuereinrichtung eine Komparatorschaltungseinheit zum Schalten von Elektrohaltemagneten umfasst.

Insofern ist es von Vorteil, wenn die Vortriebsvorrichtung mittels der Detektiereinrichtung steuerbare Elektrohaltemagnete zum Betätigen axial verschieblicher Schiebehülsen zum radialen Einrücken der Vortriebspindelmutter gegenüber der Vortriebsspindel umfasst.

Vorteilhafter Weise kann mittels der vorliegenden Erfindung trotz einer schnelleren Wechselmöglichkeit einer Infusionsspritze an einer Infusionspumpe die Gefahr einer unkontrollierten Infusion weiter signifikant reduziert werden.

Bei der vorliegenden Infusionspumpe handelt es sich insbesondere um eine Spritzenpumpe, in welcher wenigstens eine Infusionsspritze eingelegt werden kann. Die vorliegende Infusionspumpe kann sowohl als Einzelpumpe als auch in einem Ordnungssystem bzw. einer Docking-Station zusammengefasst betrieben werden.

Weitere Vorteile, Ziele und Eigenschaften vorliegender Erfindung werden anhand anliegender Zeichnung und nachfolgender Beschreibung erläutert, in welchen beispielhaft eine Antriebsvorrichtung mit einer eine Zwei-Schwellenwert-Sensoreinheit umfassenden Antriebskopfeinheit einer Infusionspumpe dargestellt und beschrieben ist. In der Zeichnung zeigen:
- Figur 1: schematisch eine Ansicht einer Infusionspumpe umfassend eine Antriebsvorrichtung mit einer Antriebskopfeinheit mit einer eine Zwei-Schwellenwert-Sensoreinheit umfassenden Detektiereinrichtung;
- Figur 2: schematisch eine längsgeschnittene Ansicht eines vorderen Bereichs der Antriebskopfeinheit;
- Figur 3: schematisch eine perspektivische teilweise geschnittene Ansicht der Antriebsvorrichtung der Infusionspumpe aus den Figuren 1 und 2 mit der Antriebskopfeinheit und mit der Vortriebseinrichtung hierfür;
- Figur 4A: schematisch eine Ansicht einer ersten Betriebsstellung der Antriebsvorrichtung aus den Figuren 1 und 2, bei welcher die Antriebskopfeinheit einer Kolbenplatte händisch zustellbar ist;
- Figur 4B: schematisch eine Ansicht einer gegenüber der ersten Betriebsstellung aus der Figur 4A fortgeschrittenen zweiten Betriebsstellung, bei welcher ein Kontaktelement der Detektiereinrichtung in der Antriebskopfeinheit einen ersten Schwellenwert der Zwei-Schwellenwert-Sensoreinheit ausgelöst hat;
- Figur 4C: schematisch eine Ansicht einer gegenüber der zweiten Betriebsstellung aus der Figur 4B fortgeschrittenen dritten Betriebsstellung, bei welcher das Kontaktelement einen zweiten Schwellenwert der Zwei-Schwellenwert-Sensoreinheit ausgelöst hat und darüber hinaus ein Drucksensor ausgelöst ist;
- Figur 5A: schematisch eine Detailansicht der Vortriebseinrichtung in der ersten Betriebsstellung hinsichtlich der Figur 4A;
- Figur 5B: schematisch eine Ansicht eines Längsschnitts der Detailansicht aus der Figur 5A;
- Figur 6A: schematisch eine Detailansicht der Vortriebseinrichtung in der zweiten Betriebsstellung hinsichtlich der Figur 4B;
- Figur 6B: schematisch eine Ansicht eines Längsschnitts der Detailansicht aus der Figur 6A;
- Figur 7A: schematisch eine Detailansicht der Vortriebseinrichtung in der dritten Betriebsstellung hinsichtlich der Figur 4C; und
- Figur 7B: schematisch eine Ansicht eines Längsschnitts der Detailansicht aus der Figur 7A.

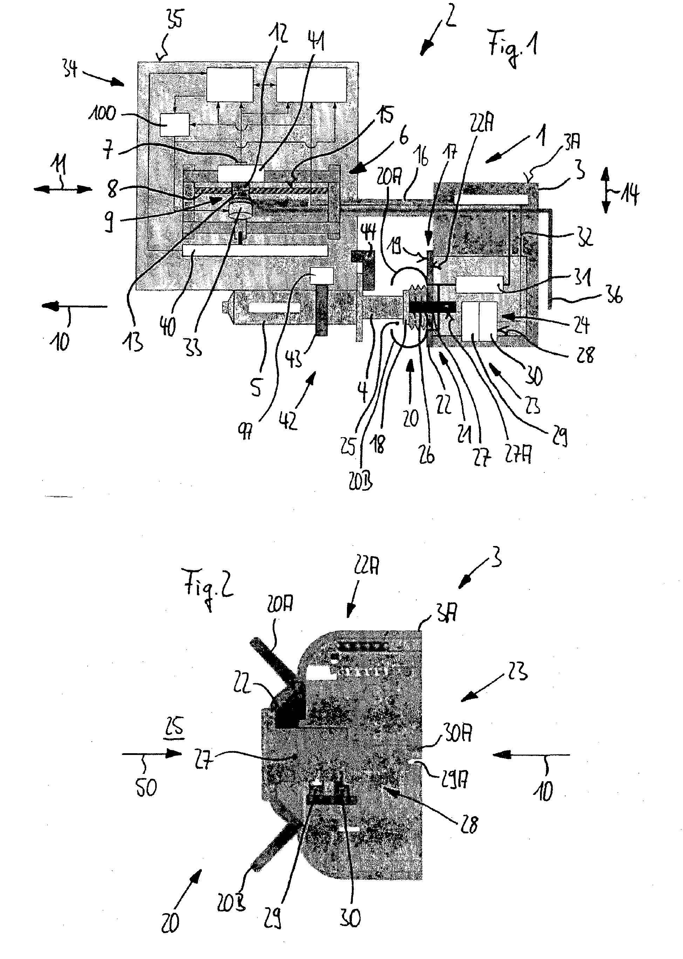

Die in den Figuren 1 bis 7 gezeigte Antriebsvorrichtung 1 einer Infusionspumpe 2 umfasst im Wesentlichen eine Antriebskopfeinheit 3 zum Bewegen eines Kolbens 4 einer an der Infusionspumpe 2 befestigten Infusionsspritze 5 und eine Vortriebseinrichtung 6 für die Antriebskopfeinheit 3.

Die Vortriebseinrichtung 6 umfasst im Wesentlichen einen Vortriebsschlitten 7, eine Vortriebsspindel 8 sowie eine mehrteilige Vortriebsspindelmutter 9. Darüber hinaus umfasst die Vortriebseinrichtung 6 noch einen Vortriebsmotor und ein entsprechendes Getriebe, welche vorliegend jedoch nicht dargestellt sind. Mit der Vortriebseinrichtung 6 wird eine lineare Zustellbewegung der Antriebskopfeinheit 3 umgesetzt. Mit dem Vortriebsschlitten 7 sind die lineare Führung und der Verdrehschutz der Antriebskopfeinheit 3 gewährleistet.

Die mehrteilige Vortriebsspindelmutter 9 bzw. der Vortriebsschlitten 7 können bei entsprechend gewählter Drehrichtung der Vortriebsspindel 8 in Kolbenantriebsrichtung 10 vorwärts angetrieben werden, wobei insbesondere die mehrteilige Vortriebsspindelmutter 9 axial entlang der Längserstreckung 11 der Vortriebsspindel 8 bewegt wird.

Die mehrteilige Vortriebsspindelmutter 9 umfasst hierbei eine erste radial bewegbare Mutterschale 12 und eine zweite radial bewegbare Mutterschale 13, wie später noch ausführlicher beschrieben ist. Jedenfalls können die radial bewegbaren Mutterschalen 12 und 13 einen axialen Formschluss mit der Vortriebsspindel 8 gewährleisten und diesen jedoch auch aufheben. Hierzu sind die beiden radial bewegbaren Mutterschalen 12 und 13 in dem Vortriebsschlitten 7 gegenüber der Vortriebsspindel 8 einrückbar bzw. ausrückbar gelagert angeordnet.

Der Begriff "radial" kennzeichnet eine radiale Bewegungsrichtung 14 im Wesentlichen quer zur Längserstreckung 11 der Vortriebsspindel 8.

Die Vortriebsspindel 8 weist ferner ein Außengewinde 15 auf, mittels welchem die mehrteilige Vortriebsspindelmutter 9 formschlüssig verbunden werden kann.

Die Antriebskopfeinheit 3 ist mittels eines Haltearms 16 an dem Vortriebsschlitten 7 befestigt, sodass diese zusammen mit dem Vortriebsschlitten 7 entsprechend axial verschieblich ist.

Die Antriebskopfeinheit 3 weist zudem eine Hauptanlageeinrichtung 17 zum Abstützen einer Kolbenplatte 18 des Infusionsspritzenkolbens 4 auf. An die Hauptanlageeinrichtung 17 stützt sich die Kolbenplatte 18 ab, wenn der Infusionsspritzenkolben 4 von der Antriebskopfeinheit 3 in Kolbenantriebsrichtung 10 geschoben wird. Die Hauptanlageeinrichtung 17 gestaltet hierfür eine Hauptanlagefläche 19 aus, an welcher sich die Kolbenplatte 18 abstützt.

Hierbei wird die Kolbenplatte 18 mittels einer Fixiereinrichtung 20 an der Antriebskopfeinheit 3 fixiert, sodass eine formschlüssige Verbindung zwischen der Kolbenplatte 18 des Infusionsspritzenkolbens 4 und der Antriebskopfeinheit 3 garantiert ist.

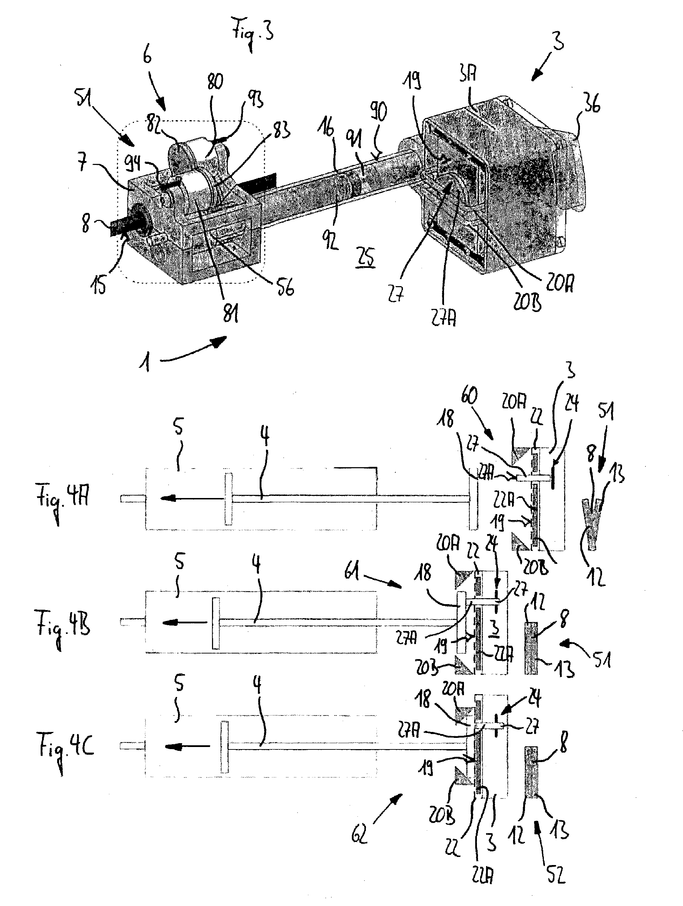

Die Fixiereinrichtung 20 ist hierbei mit Mitteln 21 zum Auslösen der Fixiereinrichtung 20 ausgestattet, welche einen Membranteller 22 umfassen, der wiederum einen Drucksensor 22A zur Verfügung stellt, mittels welchem ein Anschlagsdruck bzw. ein Anlagedruck hinsichtlich der Kolbenplatte 18 gegenüber der Hauptanlageeinrichtung 17 detektiert werden kann. Wird ein entsprechender Druck registriert, wird eine Bewegung von Fixierbügeln 20A und 20B der Fixiereinrichtung 20 ausgelöst, die dann nach innen zuschnappen und die Kolbenplatte 18 an der Hauptanlagefläche 19 form- und kraftschlüssig festlegen (siehe auch Figuren 2C und 4).

Während die Mittel 21 zum Auslösen der Fixiereinrichtung 20 insbesondere mit ihrem Membranteller 22 und ihrem Drucksensor 22A im Allgemeinen einer Detektiereinrichtung 23 der Antriebskopfeinheit 3 zugeordneter werden können, zeichnet sich die Detektiereinrichtung 23 speziell durch eine Mehr-Schwellenwert-Sensoreinheit 24 zum Detektieren des Infusionsspritzenkolbens 4 im Vorfeld 25 vor der Hauptanlageeinrichtung 17 aus.

Die Detektiereinrichtung 23 weist darüber hinaus ein Feder 26 vorgespanntes axial verlagerbares Geberelement 27 zum Beeinflussen der Mehr-Schaltschwellen-Sensoreinheit 24 auf. Das Geberelement 27 ist hierbei konstruktiv einfach direkt durch ein Kontaktelement 27A der Antriebskopfeinheit 3 ausgestaltet, welches über die Hauptanlageeinrichtung 17 hinaus bis in das Vorfeld 25 hinein ragt.

Die Mehr-Schaltschwellen-Sensoreinheit 25 zeichnet sich hierbei durch eine mindestens zweistufige Vorfelderfassungseinrichtung 28 auf, welche mit einer ersten Lichtschranke 29 und einer zweiten Lichtschranke 30 ausgerüstet ist, die axial hintereinander angeordnet sind, wodurch sie beide konstruktiv besonders einfach von dem einzigen Geberelement 27 beeinflussbar sind.

Während einerseits mittels des Drucksensors 22A des Membrantellers 22 die Funktion eines Antriebsgetriebes 31 der Fixiereinrichtung 20 gesteuert werden kann, kann andererseits mittels der Mehr-Schwellenwert-Sensoreinheit 24 die Funktion der Vortriebseinrichtung 6 beeinflusst werden, wie später noch detaillierter beschrieben ist. Hierzu ist die Mehr-Schwellenwert-Sensoreinheit 24 über eine entsprechende Signalleitung 32 mit einer Steuerungseinheit 33 der Vortriebseinrichtung 6 verbunden.

Vorteilhafter Weise umfasst die Antriebsvorrichtung 1 noch eine übergeordnete in Abhängigkeit der Detektiereinrichtung 23 arbeitende gemeinsame Regel- und Steuereinrichtung 34 zum automatischen radialen Einrücken der beiden radial bewegbaren Mutterschalen 12 und 13 an der Vortriebsspindel 8 und zum Initiieren einer mittels der radial eingerückten Vortriebsspindelmutter 9 durchführbaren Referenzfahrt der Hauptanlageeinrichtung 17 bis an Infusionsspritzenkolben 4 heran. Die gemeinsame Regel- und Steuereinrichtung 34 ist nach der Darstellung der Figur 1 im oberen Teil eines Infusionspumpengehäuses 35 der Infusionspumpe 2 angeordnet.

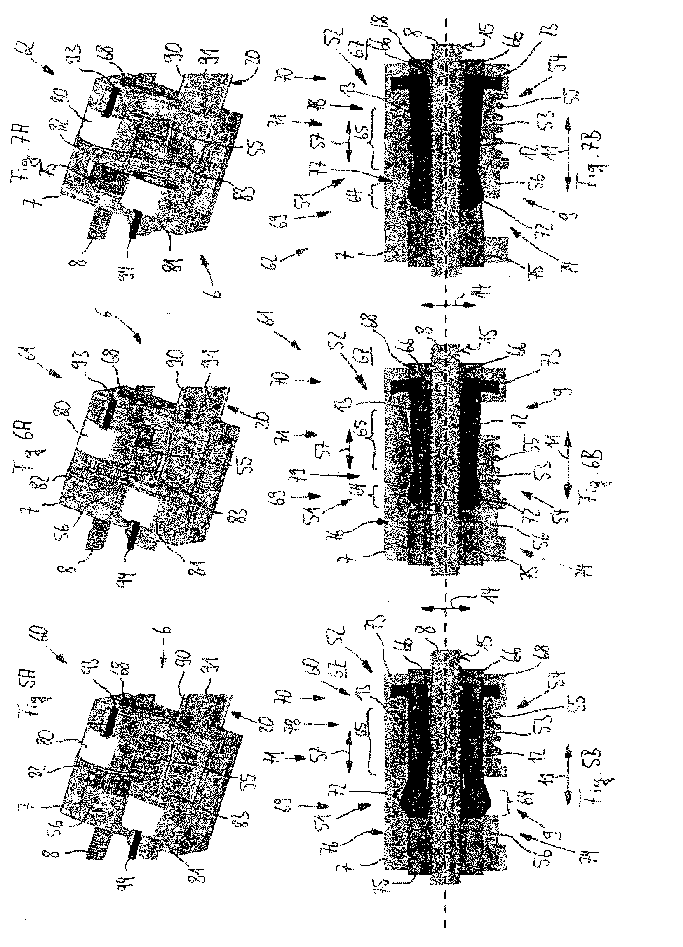

Die gemeinsame Regel- und Steuereinrichtung 34 zeichnet sich noch durch eine Komparatorschaltungseinheit 36 zum Schalten von Elektrohaltemagneten 80 bzw. 81 (siehe Figuren 4, 5A und 5B) aus, wie dies später noch detaillierter beschrieben ist.

An der Antriebskopfeinheit 3 ist noch ein manuell bedienbarer Bedienhebel 36 vorgesehen, mittels welchen die Funktionen der Fixierbügel 20A und 20B sowie der Vortriebseinrichtung 6 zusätzlich manuell betätigt werden können. Zur mechanischen Betätigung der Vortriebseinrichtung 6 mittels des Bedienhebels 36 ist dieser von der Antriebskopfeinheit 3 durch den Haltearm 16 hindurch bis zur Vortriebseinrichtung 6 geführt.

Auf die speziell in der Figur 1 schematisch illustrierte Regel- und/oder Steuereinrichtung 34 wird hier jedoch nur insoweit eingegangen, sofern deren Funktionen für die vorliegende Erfindung wesentlich sind. So umfasst die Regel- und/oder Steuereinrichtung 34 etwa noch einen Sensor 40 zum Feststellen einer korrekten Einbaulage der Infusionsspritze 5 und etwa einen Formschlusssensor 41 zum Kontrollieren eines Formschlusszustandes zwischen der mehrteiligen Vortriebsspindelmutter 9 und der Vortriebsspindel 8.

An dem Infusionspumpengehäuse 35 von außen zugänglich ist noch eine Aufnahme 42 für die Infusionsspritze 5 vorgesehen, welche einen Klemmbügel 43 zum radialen Festlegen der Infusionsspritze 5 und eine Klemmlasche 44 zum axialen Festlegen der Infusionsspritze 5 an der Aufnahme 42 der Infusionspumpe 2 umfasst.

Bei der in der Figur 2 gezeigten Ansicht ist die Antriebskopfeinheit 3 detailreicher dargestellt. Deutlich erkennbar ist die Detektiereinrichtung 23 mit ihrer Mehr-Schwellenwert-Sensoreinheit 24 und der zweistufigen Vorfelderfassungseinrichtung 28, welche speziell die zwei axial hintereinander angeordneten Lichtschranken 29 und 30 und das hierzu einzig erforderliche Geberelement 27 zum Ansprechen der beiden Lichtschranken 29 bzw. 30 aufweist.

Die erste Lichtschranke 29 ist mittels einer ersten elektrischen Kabelverbindung 29A der Signalleitung 32 (siehe Figur 1) und die zweite Lichtschranke 30 ist mittels einer zweiten elektrischen Kabelverbindung 30A der Signalleitung 32 entsprechend mit der Regel- und/oder Steuereinrichtung 34 elektrisch verbunden.

Das Geberelement 27 ist in dieser Darstellung aufgrund eines Kontaktes mit der hier nicht gezeigten Kolbenplatte 18 gemäß der Pfeilrichtung 50 derart weit in die Antriebskopfeinheit 3 hinein verlagert, dass es bereits die zweite Lichtschranke 30 unterbrochen hat. Insofern befindet sich die Antriebsvorrichtung 1 in der zweiten Betriebsstellung 61, welche später noch genauer erläutert ist.

Deutlich ist das in dem Membranteller 22 beweglich integrierte Geberelement 27 der zweistufigen Vorfelderkennungseinrichtung 28 zu erkennen, welches relativ verschieblich zu dem Membranteller 22 gelagert ist.

Da der hinter dem Membranteller 22 angeordnete Drucksensor 22A noch nicht ausreichend stark von der Kolbenplatte 18 gedrückt ist, befinden sich die beiden Fixierbügel 20A und 20B der Fixiereinrichtung 20 noch in der geöffneten Stellung (siehe auch Figur 4B).

Im Folgenden werden die funktionalen Zusammenhänge der vorliegenden Antriebsvorrichtung 1 speziell hinsichtlich der Figuren 4, 5, 6 und 7 näher erläutert. Die Antriebsvorrichtung 1 umfasst eine mittels der Mehr-Schwellenwert-Sensoreinheit 24 automatisch auslösbare Blockiereinrichtung 51 zum Blockieren einer händischen Zustellbewegung der Antriebskopfeinheit 3 in Kolbenantriebsrichtung 10, wobei die Blockiereinrichtung 51 vorteilhafter Weise direkt die mehrteilige Vortriebsspindelmutter 9 umfasst.

Die Blockiereinrichtung 51 weist ein axial verschiebliches Betätigungselement 52 auf, welches hier beispielhaft zumindest eine axial verschiebliche Auslösehülse 53 umfasst, die konzentrisch um die beiden Mutterschalen 12 und 13 der Vortriebsspindelmutter 9 herum angeordnet ist. Die Auslösehülse 53 ist hierbei in Längserstreckung 11 der Vortriebsspindel 8 verschieblich radial außen an der Vortriebsspindelmutter 9 gelagert.

Damit die Blockiereinrichtung 51 extrem schnell aktiviert werden kann, verfügt sie über Mittel 54 zum Beschleunigen der Auslösehülse 53, wobei die Beschleunigungsmittel 54 konstruktiv einfach durch eine Spiralfeder 55 ausgestaltet sind, die wiederum radial außen an der Auslösehülse 53 gelagert ist.

Darüber hinaus verfügt das Betätigungselement 52 noch über eine Freigabehülse 56, die ebenfalls axial verschieblich und konzentrisch um die beiden Mutterschalen 12 und 13 der Vortriebsspindelmutter 9 herum angeordnet sein kann.

Insofern sind beide Schiebehülsen 53 und 56 auch axial in Bezug auf die Längserstreckung 57 der beiden radial bewegbaren Mutterschalen 12 und 13 verschieblich gelagert.

Zum besseren Verständnis der Funktionsweise des vorliegenden Betätigungselements 52 der Vortriebsspindelmutter 9 im Zusammenhand mit der Funktion der Mehr-Schwellenwert-Sensoreinheit 24 ist in den Figuren 4A, 5A und 5B beispielhaft eine erste Betriebsstellung 60 der Antriebsvorrichtung 1 gezeigt, bei welcher die beiden Mutterschalen 12 und 13 sich nicht im formschlüssigen Eingriff mit dem Außengewinde 15 der Vortriebsspindel 8 befinden. In dieser ersten Betriebsstellung 60 kann die Antriebskopfeinheit 3 mittels einer händischen Zustellbewegung in Kolbenantriebsrichtung 10 schneller manuell an die Kolbenplatte 18 heran bewegt werden (siehe auch Figur 4A). Hierbei befindet sich das Geberelement 27 noch vor den beiden Lichtschranken 29 und 30. Insofern ist die Vortriebsspindelmutter 9 noch geöffnet, wie dies auch in der Darstellung nach der Figur 4A rechts außen schematisch dargestellt ist. Diese Betriebsstellung 60 kann insbesondere dann gewählt werden, wenn ein Infusionsspritzenwechsel an der Infusionspumpe 2 vorgenommen wird.

Bei einer in den Figuren 4B, 6A und 6B beispielhaft gezeigten zweiten Betriebsstellung 61 ist das Geberelement 27 bereits bis in die erste Lichtschranke 29 hinein verlagert, wodurch die Blockiereinrichtung 51 ausgelöst ist. Die Vortriebseinrichtung 6 ist jetzt durch die radial eingerückte Vortriebsspindelmutter 9 an sich blockiert, sodass eine händische Zustellbewegung der Antriebskopfeinheit 3 in Kolbenantriebsrichtung 10 weiter nicht möglich ist. Insofern wird hierdurch die Gefahr ausgeschlossen, dass der Membranteller 22 kritisch an die Kolbenplatte 18 anstößt und hierdurch versehentlich eine für einen Patienten wohlmöglich gefährliche Bolusgabe erfolgt. Die Kolbenplatte 18 drückt noch nicht auf den Drucksensor 22A hinter dem Membranteller 22, sodass die Fixierbügel 20A und 20B noch geöffnet sind.

Letztlich befindet sich bei einer weiteren hinsichtlich der Figuren 4C, 7A und 7B beispielhaft ausgewählten dritten Betriebsstellung 62 die Antriebsvorrichtung 1 in einem Grundbetrieb, bei welchem der Drucksensor 22A ausreichend stark von der Kolbenplatte 18 gedrückt ist, wodurch die Fixierbügel 20A und 20B geschlossen sind. Die Antriebskopfeinheit 3 wird nun durch die Vortriebseinrichtung 6 sukzessive in Kolbenantriebsrichtung 10 angetrieben. Insofern kann eine gewünschte Infusionstherapie in an sich bekannter Weise mittels der Infusionspumpe 2 erfolgen.

Betrachtet man nun die Darstellungen aller Figuren 5, 6 und 7 ist gut zu erkennen, dass die beiden radial bewegbaren Mutterschalen 12 und 13 jeweils einen Eingreifbereich 64 zum Eingreifen in das Außengewinde 14 der Vortriebsspindel 8 aufweisen. Insofern kann eine Formschlussverbindung zwischen den Mutterschalen 12 und 13 der Vortriebsspindelmutter 9 und dem Außengewinde 15 der Vortriebsspindel 8 über dem jeweiligen Eingreifbereich 64 der Mutterschalen 12 bzw. 13 hergestellt werden, wenn dieser Eingreifbereich 64 ordnungsgemäß radial in Richtung der Vortriebsspindel 8 bewegt wird bzw. gehalten wird. Die beiden Mutterschalen 12, 13 sind dann gegenüber der Vortriebsspindel 8 eingerückt. Dies ist beispielsweise bei den beiden Betriebsstellungen 61 und 62 der Fall. Darüber hinaus weisen die beiden radial bewegbaren Mutterschalen 12 und 13 noch einen Auflagebereich 65 auf, mittels welchen die Mutterschalen 12 und 13 höchstens radial auf das Außengewinde 15 aufliegen können, jedoch nicht mit diesem in Eingriff gelangen können. Der Eingreifbereich 64 und der Auflagebereich 65 sind axial hintereinander angeordnet.

Um die beiden radial bewegbaren Mutterschalen 12, 13 jeweils mit den beiden Schiebehülsen 53, 56 gegenüber der Vortriebsspindel 8 um eine Kippachse 66, welche im Wesentlichen quer zu Längserstreckung 57 der jeweiligen Mutterschale 12 bzw. 13 verläuft, herum radial anstellen zu können, sind die beiden Mutterschalen 12 und 13 endseitig 67 jeweils auf einem Vortriebsschlittenlager 68 gelagert. Die Kippachse 66 erstreckt sich nach der Darstellung der Figuren 5B, 6B und 7B senkrecht zur Papierebene.

Darüber hinaus weist die Vortriebsspindelmutter 9 einen Kopfbereich 69 und einen Fußbereich 70 auf, deren jeweilige Außendurchmesser größer sind als ein dazwischen angeordnetes Vortriebsspindelmuttergebiet 71, wobei der Kopfbereich 69 eine Rampe 72 zum Aufschieben für die beiden axial verschieblichen Schiebehülsen 53 und 56 und der Fußbereich 70 ein Gegenlager 73 für die beiden axial verschieblichen Schiebehülsen 53 und 56 ausgestaltet.

An einem dem Vortriebsschlittenlager 68 gegenüberliegenden Vortriebsspindelmutterende 74 befindet sich eine an dem Vortriebsschlitten 7 gelagerte Parkeinrichtung 75 für die Freigabehülse 56, wobei die Parkeinrichtung 75 axial neben der Vortriebsspindelmutter 9 platziert ist. Ist die Freigabehülse 56 auf den Sitz der Parkeinrichtung 75 aufgeschoben und hierbei neben der Vortriebsspindelmutter 9 geparkt, befindet sich die Freigabehülse 56 in einer Öffenposition 76. Dies ist bei den beiden Betriebsstellungen 60 und 61 der Fall. Bei der dritten Betriebsstellung 62 ist die Freigabehülse 56 auf die Rampe 72 verschoben und sie befindet sich dementsprechend in einer Schließposition 77.

Die Auslösehülse 53 befindet sich in den beiden Betriebsstellungen 60 und 62 jeweils in ihrer eigenen Öffenposition 78, in welcher die Auslösehülse 53 dem Gegenlager 73 näher als der Rampe 72 zugewandt ist. Lediglich bei der Betriebsstellung 61 befindet sich die Auslösehülse 53 in einer ihr zugeteilten Schließposition 79.

Des Weiteren umfasst die Vortriebseinrichtung 8 ein erstes Elektrohaltemagnet 80 und ein zweites Elektrohaltemagnet 81, welche in diesem Ausführungsbeispiel oberhalb des Vortriebsschlittens 7 angeordnet sind.

Der erste Elektrohaltemagnet 80 ist hierbei der Auslösehülse 53 zugeordnet und er kann dementsprechend mit einem Magnetteller 82 der Auslösehülse 53 korrespondieren. Hierzu ist der Magnetteller 82 vor dem ersten Elektrohaltemagnet 80 platziert und gekoppelt mit der Auslösehülse 53 axial verschieblich gelagert. Bei den beiden Betriebsstellungen 60 und 62 ist der erste Elektrohaltemagnet 80 bestromt, sodass er mit dem Magnetteller 82 Kontakt halten und damit auch die Auslösehülse 53 in ihrer Öffenposition 78 halten kann. Bei der zweiten Betriebsstellung 61 ist der Magnetteller 82 von dem ersten unbestromten Elektrohaltemagneten 80 beabstandet.

Der zweite Elektrohaltemagnet 81 ist dementsprechend der Freigabehülse 56 zugeordnet und er kann mit einem Magnetteller 83 der Freigabehülse 56 korrespondieren, wobei dieser Magnetteller 83 hierzu vor dem zweiten Elektrohaltemagnet 81 platziert ist. Der Magnetteller 83 wird gemeinsam mit der Freigabehülse 56 axial verschoben, da er mit dieser gekoppelt ist. Insofern befindet sich der Magnetteller 83 bei den Betriebsstellungen 60 und 61 in Kontakt mit dem zweiten bestromten Elektrohaltemagnet 81, während er bei der zweiten Betriebsstellung 62 von dem unbestromten zweiten Elektrohaltemagnet 81 entfernt angeordnet ist.

Ist nun ein Infusionsspritzenwechsel gewünscht bzw. erforderlich, wird die Infusion gestoppt. Anschließend wird der Bedienhebel 36 an der Antriebskopfeinheit 3 betätigt. Mit dem Betätigen des Bedienhebels 36 wird über den Haltearm 16, welcher als Rohr 90 (siehe Figur 3) konzipiert ist, via einer inneren Fixierungswelle 91 die Feder 92 belastete Freigabehülse 56 in Richtung des zweiten Elektrohaltemagneten 81 bewegt. Dieses Betätigen wird zusätzlich durch einen einen hier nicht gezeigten Sensor umfassenden Mikroschalter ermittelt, wodurch die beiden Elektrohaltemagnete 80 und 81 über entsprechende Elektrokabel 93 bzw. 94 bestromt und die beiden Schiebehülsen 53 und 56 infolgedessen voneinander getrennt werden. Dabei öffnen sich die beiden Mutterschalen 12 und 13. Auch die Fixierbügel 20A bzw. 20B der Fixiereinrichtung 20 öffnen sich aufgrund der Betätigung des Bedienhebels 36. Der Formschluss zwischen der Vortriebsspindel 8 und der Vortriebsspindelmutter 9 ist gemäß der ersten Betriebsstellung 60 aufgehoben und die Antriebskopfeinheit 3 kann entgegen der Kolbenantriebsrichtung 10 in eine ausgefahrene Stellung (siehe Figur 4A) gebracht werden.

Anschließend wird der Klemmbügel 43 geöffnet und entsprechend verschwenkt, sodass die Aufnahme 42 gut zugänglich ist. Ein im Inneren des Infusionspumpengehäuses 35 montiertes Potentiometer 97 erkennt den Zustand des Klemmbügels 43 (siehe Figur 1). Jetzt kann die auszuwechselnde Infusionsspritze entnommen und die neue Infusionsspritze 5 in die Aufnahme 42 eingelegt werden. Die Infusionsspritzenflügel der einzulegenden Infusionsspritze 5 werden hierbei an der Klemmlasche 44 festgelegt, sodass die neue Infusionsspritze 5 an der Infusionsspritzenpumpe 2 axial fixiert ist. In diesem Zusammenhang wird auch der Klemmbügel 43 wieder geschlossen, sodass die Infusionsspritze 5 auch radial fixiert ist. Mittels des Potentiometers 97 am Klemmbügel 43 wird sogleich der Infusionsspritzendurchmesser gemessen.

Nun kann die Antriebskopfeinheit 3 entsprechend des Füllstands der Infusionsspritze 5 und damit auch der Position des Infusionsspritzenkolbens 4 an die Kolbenplatte 18 heran geführt werden. Damit hierbei größere Abstände zwischen der Antriebskopfeinheit 3 und der Kolbenplatte 18 schneller überwunden werden können, kann dies durch eine händische Zustellbewegung in Kolbenantriebsrichtung 10 geschehen, da die beiden radial bewegbaren Mutterschalen 12 und 13 sich nicht im Eingriff mit dem Außengewinde 15 der Vortriebsspindel 8 befinden, wie dies bei der ersten Betriebsstellung 60 gemäß der Figuren 4A, 5A und 5B dargestellt ist. Bei der ersten Betriebsstellung 60 sind die beiden Elektrohaltemagnete 80 und 81 bestromt, die Schiebehülsen 53 und 56 voneinander getrennt angeordnet, die beiden Mutterschalen 12 und 13 radial geöffnet. Außerdem ist hierbei die Fixiereinrichtung 20 an der Antriebskopfeinheit 3 geöffnet.

Sobald jedoch das Kontaktelement 27A mit der Kolbenplatte 22 in Kontakt tritt (siehe Figur 4B), bewegt sich insbesondere das Geberelement 27 nach innen in die Antriebskopfeinheit 3 hinein. Im Inneren des Antriebskopfeinheitsgehäuses 3A durchfährt das Geberelement 27 nacheinander die zwei Lichtschranken 29 und 30 der Mehr-Schaltschwellen-Sensoreinheit 24.

Beim Durchfahren der ersten Lichtschranke 29 (siehe weiter Figur 4B) wird die Bestromung des ersten Elektrohaltemagneten 80 unterbrochen und die Auslösehülse 53 schnellt mittels der weiteren Feder 92 über die Rampen 72 der beiden radial bewegbaren Mutterschalen 12 und 13, wodurch die Vortriebsspindelmutter 9 mit den Eingreifbereichen 64 schlagartig in das Außengewinde 15 der Vortriebsspindel 8 formschlüssig eingreift. Hierbei blockiert die Vortriebseinrichtung 6, wie dies bei der zweiten Betriebsstellung 61 gemäß der Figuren 4B, 6A und 6B dargestellt ist. Eine händische Zustellbewegung in Kolbenantriebsrichtung 10 wird hierdurch schlagartig unterbunden.

Jedoch fährt die Vortriebseinrichtung 6 jetzt automatisch weiter, bis das Geberelement 27 die zweite Lichtschranke 30 durchfährt (siehe insbesondere Figur 4C). Die zweite Lichtschranke 30 ist hierbei notwendig, da die Positionsveränderung der radial geschlossenen und somit radial eingerückten Mutterschalen 12 und 13 mit ihren Eingreifbereichen 64 gegenüber der Vortriebsspindel 8 abhängig ist, von der Reaktionsgeschwindigkeit des ersten Elektrohaltemagneten 80 und der Bewegungsgeschwindigkeit der Antriebskopfeinheit 3 bis zur Kolbenplatte 18. Weiterhin besteht die Möglichkeit, dass die beiden radial bewegbaren Mutterschalen 12 und 13 beim Schließen der Vortriebsspindelmutter 9 lediglich auf den Gewindeflanken des Außengewindes 15 aufliegen und der Eingreifbereich 64 der Vortriebsspindelmutter 9 somit nicht wirkungsvoll in das Außengewinde 15 eingerückt ist. Jedoch kann durch ein kurzes Verfahren bis zur zweiten Lichtschranke 30 die Vortriebsspindelmutter 9 ordnungsgemäß in die Vortriebsspindel 8 eingreifen. Mit dem Erreichen der zweiten Lichtschranke 30 ist die lineare Position der Antriebsvorrichtung 1 dann definiert.

Bei dieser Referenzfahrt im Sinne der Erfindung befindet sich die Vortriebseinrichtung 6 in der zweiten Betriebsstellung 61, bei welcher der erste Elektrohaltemagnet 80 stromlos, der zweite Elektrohaltemagnet 81 jedoch bestromt ist. Beide Schiebehülsen 53 und 56 sind im Vortriebsschlitten 7 nach links verschoben und die beiden Mutterschalen 12 und 13 sind radial geschlossen. Die Fixiereinrichtung 20 an der Antriebskopfeinheit 3 ist noch geöffnet (siehe Figur 4B).

Die Antriebskopfeinheit 3 fährt den abgeglichenen Weg von der zweiten Lichtschranke 30 bis zum Membranteller 22 und dem dahinter angeordneten Drucksensor 22A ab, mittels welchem ein korrektes Anliegen der Kolbenplatte 18 an der Hauptanlagefläche 19 an der Antriebskopfeinheit 3 detektiert und signalisiert wird (siehe Figur 4C).

Der zweite Elektrohaltemagnet 81 wird stromlos geschaltet, die Freigabehülse 56 verlagert sich in ihre Schließposition 77 (siehe insbesondere Figuren 7A und 7B), die Fixiereinrichtung 20 schließt sich und die Fixierbügel 20A und 20B legen sich form- und kraftschlüssig an die Kolbenplatte 18 der Infusionsspritze 5 an. Die Infusionsspritze 5 ist nun sicher und spielfrei gefasst (siehe Figur 4C).

Mittels des Klemmbügels 43 und dem Potentiometer 97 ist bereits der Infusionsspritzendurchmesser gemessen worden, sodass in einem Display (hier nicht gezeigt) der Infusionspumpe 2 eine Auswahl an entsprechenden Infusionsspritzen 5 vorgeschlagen wird. Nach einer Bestätigung der gültigen Infusionsspritze 5 kann die gewünschte Infusionstherapie eingegeben werden und es kann nun die Infusion gestartet werden, wobei sich die Antriebskopfeinheit 3 und die Vortriebseinrichtung 6 in der dritten Betriebsstellung 62 gemäß der Figuren 4C, 7A und 7B befindet. In der dritten Betriebsstellung 62 ist die Vortriebseinrichtung 6 somit verriegelt. Dies bedeutet, dass die beiden Elektrohaltemagnete 80, 81 stromlos sind.

Besonders vorteilhaft ist es, dass hierbei ein energieloser Zustand hinsichtlich der beiden Elektrohaltemagnete 80, 81 vorliegt, sodass ein Funktionieren der dritten Betriebsstellung 62 auch ohne Bestromen der Elektrohaltemagnete 80, 81 gewährleistet werden kann. Die beiden Schiebehülsen 53, 56 sind innerhalb des Vortriebsschlittens 7 nach rechts verschoben, da die weitere Feder 92 stärker ausgelegt ist als die Spiralfeder 55. Die beiden Mutterschalen 12 und 13 bleiben radial verschlossen sowie auch die Fixiereinrichtung 23 an der Antriebskopfeinheit 3 verschlossen ist.

Damit die Elektrohaltemagnete 80 und 81 besonders schnell stromlos geschaltet werden können, umfasst die Antriebsvorrichtung 1 vorteilhafter Weise noch eine Komparatorschaltungseinheit 100 (siehe Figur 100). Da das jeweilige Magnetfeld der Elektrohaltemagnete 80 und 81 mit Hilfe der Bestromung erzeugt wird, ist es vorteilhaft, wenn für ein schnelles Abklingen der Bestromung eine kurzzeitige Überspannung zugelassen wird. Mittels der Komparatorschaltungseinheit 100 wird ein besonders rasches Abfallen des Energiezustands an dem jeweiligen Elektrohaltemagnet 80 bzw. 81 erzielt, sodass sich die Reaktionszeit zum Blockieren der Vortriebseinrichtung 6 weiter verringern lässt.

Es versteht sich, dass es sich bei dem vorstehend erläuterten Ausführungsbeispiel lediglich um eine erste Ausgestaltung der erfindungsgemäßen Antriebsvorrichtung handelt. Insofern beschränkt sich die Ausgestaltung der Erfindung nicht auf dieses Ausführungsbeispiel.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Antriebsvorrichtung
- 2: Infusionspumpe
- 3: Antriebskopfeinheit
- 3A: Antriebskopfeinheitsgehäuse
- 4: Infusionsspritzenkolben
- 5: Infusionsspritze
- 6: Vortriebseinrichtung
- 7: Vortriebsschlitten
- 8: Vortriebsspindel
- 9: mehrteilige Vortriebsspindelmutter
- 10: Kolbenantriebsrichtung
- 11: Längserstreckung
- 12: erste Mutterschale
- 13: zweite Mutterschale
- 14: radiale Bewegungsrichtung
- 15: Außengewinde
- 16: Haltearm
- 17: Hauptanlageeinrichtung
- 18: Kolbenplatte
- 19: Hauptanlagefläche
- 20: Fixiereinrichtung
- 20A: erster Fixierbügel
- 20B: zweiter Fixierbügel
- 21: Mittel zum Auslösen
- 22: Membranteller
- 22A: Drucksensor
- 23: Detektiereinrichtung
- 24: Mehr-Schwellenwert-Sensoreinheit
- 25: Vorfeld
- 26: Feder
- 27: Geberelement
- 27A: Kontaktelement
- 28: Vorfelderfassungseinrichtung
- 29: erste Lichtschranke
- 29A: erste Kabelverbindung
- 30: zweite Lichtschranke
- 30A: zweite Kabelverbindung
- 31: Antriebsgetriebe
- 32: Signalleitung
- 33: Steuerungseinheit
- 34: gemeinsame Regel- und/oder Steuereinrichtung
- 35: Infusionspumpengehäuse
- 36: Bedienhebel
- 40: Sensor zum Feststellen
- 41: Formschlusssensor
- 42: Aufnahme
- 43: Klemmbügel
- 44: Klemmlasche
- 50: Pfeilrichtung
- 51: Blockiereinrichtung
- 52: Betätigungselement
- 53: Auslösehülse
- 54: Beschleunigungsmittel
- 55: Spiralfeder bzw. Auslösehülsefeder
- 56: Freigabehülse
- 57: Längserstreckung
- 60: erste Betriebsstellung
- 61: zweite Betriebsstellung
- 62: dritte Betriebsstellung
- 64: Eingreifbereich
- 65: Auflagebereich
- 66: Kippachse
- 68: Vortriebsschlittenlager
- 69: Kopfbereich
- 70: Fußbereich
- 71: Vortriebsspindelmuttergebiet
- 72: Rampe
- 73: Gegenlager
- 74: Vortriebsspindelmutterende
- 75: Parkeinrichtung
- 76: Öffenposition
- 77: Schließposition
- 78: weitere Öffenposition
- 79: weitere Schließposition
- 80: erster Elektrohaltemagnet bzw. Auslösehülsemagnet
- 81: zweiter Elektrohaltemagnet bzw. Freigabehülsemagnet
- 82: erster Magnetteller
- 83: zweiter Magnetteller
- 90: Rohr
- 91: Fixierungswelle
- 92: weitere Feder bzw. Freigabehülsefeder
- 93: erstes Elektrokabel
- 94: zweites Elektrokabel
- 97: Potentiometer
- 100: Komparatorschaltungseinheit

## Patentansprüche

1. Antriebsvorrichtung (1) zum linearen Bewegen eines Infusionsspritzenkolbens (4) einer an einer Infusionspumpe (2) angeordneten Infusionsspritze (5) mit einer Antriebskopfeinheit (3) umfassend eine Hauptanlageeinrichtung (17) zum Abstützen des Infusionsspritzenkolbens (4), eine Fixiereinrichtung (20) zum Festlegen des Infusionsspritzenkolbens (4) an der Hauptanlageeinrichtung (17) sowie Mittel (21) zum Auslösen der Fixiereinrichtung (20) und mit einer Vortriebseinrichtung (6) für die Antriebskopfeinheit (1) umfassend eine motorisch antreibbare Vortriebsspindel (8) und eine wenigstens eine radial bewegbare Mutterschale (12, 13) aufweisende mehrteilige Vortriebsspindelmutter (9), wobei die Antriebskopfeinheit (3) zusätzlich eine Detektiereinrichtung (23) zum Detektieren des Infusionsspritzenkolbens (4) vor der Hauptanlageeinrichtung (17) umfasst,
**dadurch gekennzeichnet, dass**
die Detektiereinrichtung (23) eine Mehr-Schwellenwert-Sensoreinheit (24) mit einer mindestens zweistufigen Vorfelderfassungseinrichtung (28) zum Erfassen unterschiedlicher Positionen des Infusionsspritzenkolbens (4), bevor eine Kolbenplatte (18) des Infusionsspritzenkolbens (4) mit der Hauptanlageeinrichtung (17) in Kontakt kommt, aufweist.

2. Antriebsvorrichtung (1) nach Anspruch 1,
**dadurch gekennzeichnet, dass**
die Mehr-Schwellenwert-Sensoreinheit (24) wenigstens zwei mittels eines einzigen Geberelementes (27) auslösbare Sensoren (29, 30) aufweist, wobei das Geberelement (27) ein über die Hauptanlageeinrichtung (17) hinausragendes Kontaktelement (27A) umfasst.

3. Antriebsvorrichtung (1) nach Anspruch 1 oder 2,
**dadurch gekennzeichnet, dass**
die Detektiereinrichtung (23) wenigstens zwei Lichtschranken (29, 30) umfasst, die axial hintereinander innerhalb der Antriebskopfeinheit (3) angeordnet sind.

4. Antriebsvorrichtung (1) nach Anspruch 3,
**dadurch gekennzeichnet, dass**
wenigstens eine der beiden Lichtschranken (29, 30) als Gabellichtschranke ausgestaltet ist.

5. Antriebsvorrichtung (1) nach einem der Ansprüche 1 bis 4,
**dadurch gekennzeichnet, dass**
die Hauptanlageeinrichtung (17) einen Membranteller (22) aufweist, welcher ein ein Kontaktelement (27A) umfassendes Geberelement (27) zum Schalten von Sensoren (29, 30) der Mehr-Schwellenwert-Sensoreinheit (24) umfasst.

6. Antriebsvorrichtung (1) nach einem der Ansprüche 1 bis 5,
**dadurch gekennzeichnet, dass**
ein einziges Geberelement (27) der Mehr-Schwellenwert-Sensoreinheit (24) innerhalb der Mittel (21) zum Auslösen der Fixiereinrichtung (20) angeordnet ist.

7. Antriebsvorrichtung (1) nach einem der Ansprüche 1 bis 6,
**dadurch gekennzeichnet, dass**
ein einziges Geberelement (27) der Mehr-Schwellenwert-Sensoreinheit (24) einen Vorlaufweg mit einem Wert zwischen 2 mm und 10 mm, vorzugsweise mit einem Wert von 4 mm, aufweist, bevor eine erste Stufe der zweistufigen Vorfelderfassungseinrichtung (28) von dem einzigen Geberelement (27) auslösbar ist.

8. Antriebsvorrichtung (1) nach einem der Ansprüche 1 bis 7,
**dadurch gekennzeichnet, dass**
die Antriebsvorrichtung (1) wenigstens eine in Abhängigkeit der Detektiereinrichtung (23) arbeitende gemeinsame Regel- und/oder Steuereinrichtung (34) zum automatischen radialen Einrücken der wenigstens einen radial bewegbaren Mutterschale (12, 13) an der Vortriebsspindel (8) und zum Initiieren einer mittels der radial eingerückten Vortriebsspindelmutter (9) durchführbaren Referenzfahrt der Hauptanlageeinrichtung (17) bis an den Infusionsspritzenkolben (4) heran aufweist.

9. Antriebsvorrichtung (1) nach Anspruch 8,
**dadurch gekennzeichnet, dass**
die gemeinsame Regel-und/oder Steuereinrichtung (34) eine Komparatorschaltungseinheit (100) zum Schalten von Elektrohaltemagneten (80, 81) umfasst.

10. Antriebsvorrichtung (1) nach einem der Ansprüche 1 bis 9,
**dadurch gekennzeichnet, dass**
die Vortriebseinrichtung (6) mittels der Detektiereinrichtung (23) steuerbare Elektrohaltemagnete (80, 81) zum Betätigen axial verschieblicher Schiebehülsen (53, 56) zum radialen Einrücken der Vortriebspindelmutter (9) gegenüber der Vortriebsspindel (8) umfasst.

11. Infusionspumpe (2) mit einer Antriebskopfeinheit (3) zum Bewegen eines Infusionsspritzenkolbens (4) einer an der Infusionspumpe (2) angeordneten Infusionsspritze (5) und mit einer Vortriebseinrichtung (6) zum Treiben der Antriebkopfeinheit (3),
**gekennzeichnet durch**
eine Antriebsvorrichtung (1) nach einem der vorhergehenden Ansprüche.

12. Verfahren zur Inbetriebnahme einer Infusionsspritze (5) an einer Infusionspumpe (2) mit einer Antriebskopfeinheit (3) zum linearen Bewegen eines Infusionsspritzenkolbens (4), bei welchem wenigstens eine radial bewegbare Mutterschale (12, 13) einer Vortriebsspindelmutter (9) radial von einer Vortriebsspindel (8) einer Vortriebseinrichtung (6) zum Treiben der Antriebskopfeinheit (3) radial ausgerückt wird, sodass die Antriebskopfeinheit (3) einer Kolbenplatte (18) des Infusionsspritzenkolbens (4) händisch schnell axial zugestellt werden kann, und bei welchem zum Verhindern einer unbeabsichtigten Bolusgabe die Kolbenplatte (18) hierbei mit einer der Antriebskopfeinheit (3) zugeordneten Detektiereinrichtung (23) mit einer Mehr-Schwellenwert-Sensoreinheit (24) und eines mindestens zweistufigen Vorfelderfassungseinrichtung (28) detektiert werden kann, bevor die Kolbenplatte (18) mit einer Hauptanlagefläche (19) einer Hauptanlageeinrichtung (17) der Antriebskopfeinheit (3) in Kontakt kommt, wobei
• in einem ersten Detektierschritt ein Kontaktelement (27A) der Detektiereinrichtung (23) ein erstes Sensorelement (29) auslöst, wodurch das händische Zustellen der Antriebskopfeinheit (3) mittels der Vortriebsspindelmutter (9) und der Vortriebsspindel (8) blockiert wird, indem die wenigstens eine radial bewegbare Mutterschale (12, 13) radial an der Vortriebsspindel (8) eingerückt wird, wobei anschließend mittels der Vortriebseinrichtung (6) die Antriebskopfeinheit (3) der Kolbenplatte (18) motorgetrieben weiter zugestellt wird, dass
• in einem zweiten Detektierschritt das Kontaktelement (27A) ein zweites Sensorelement (30) auslöst, wodurch eine motorgetriebene Referenzfahrt der Antriebskopfeinheit (3) bis an die Hauptanlagefläche (19) der Hauptanlageeinrichtung (17) heran initiiert wird, und dass
• in einem dritten Detektierschritt im Zuge eines Kontakts der Kolbenplatte (18) mit der Hauptanlagefläche (19) die motorgetriebene Referenzfahrt gestoppt wird und Haltebügel (20A, 20B) einer Fixiereinrichtung (20) für die Kolbenplatte (18) geschlossen werden, wodurch die Kolbenplatte (18) an der Hauptanlageeinrichtung (17) festgelegt wird.

13. Verfahren nach Anspruch 12,
**dadurch gekennzeichnet, dass**
die händische Zustellung der Antriebskopfeinheit (3) blockiert wird, bevor das Kontaktelement (27A) das zweite Sensorelement (30) auslöst.

14. Verfahren nach Anspruch 12 oder 13,
**dadurch gekennzeichnet, dass**
die motorgetriebene Referenzfahrt anhand einer definierten Anzahl an Motorschritten durchgeführt wird.

## Claims

1. A drive device (1) for linear movement of an infusion syringe piston (4) of an infusion syringe (5) arranged at an infusion pump (2) comprising a drive head unit (3) including a main bearing means (17) for supporting the infusion syringe piston (4), a fixing means (20) for fixing the infusion syringe piston (4) on the main bearing means (17) as well as means (21) for triggering the fixing means (20) and comprising an advancing means (6) for the drive head unit (1) including a motor-drivable advancing spindle (8) and a multi-part advancing spindle nut (9) including at least one radially movable nut shell (12, 13), wherein the drive head unit (3) in addition comprises detecting means (23) for detecting the infusion syringe piston (4) ahead of the main bearing means (17),
**characterized in that**
the detecting means (23) includes a multi-threshold sensor unit (24) having an at least two-stage upstream detection means (28) for detecting different positions of the infusion syringe piston (4) before a piston plate (18) of the infusion syringe piston (4) enters into contact with the main bearing means (17).

2. The drive device (1) according to claim 1,
**characterized in that**
the multi-threshold sensor unit (24) includes at least two sensors (29, 30) to be triggered by means of one single transmitter element (27), said transmitter element (27) comprising a contact element (27A) projecting from the main bearing means (17).

3. The drive device (1) according to claim 1 or 2,
**characterized in that**
the detecting means (23) comprises at least two light barriers (29, 30) which are arranged axially in series within the drive head unit (3).

4. The drive device (1) according to claim 3,
**characterized in that**
at least either of the two light barriers (29, 30) is a fork light barrier.

5. The drive device (1) according to any one of the claims 1 to 4,
**characterized in that**
the main bearing means (17) includes a diaphragm plate (22) comprising a transmitter element (27) including a contact element (27A) for switching sensors (29, 30) of the multi-threshold sensor unit (24).

6. The drive device (1) according to any one of the claims 1 to 5,
**characterized in that**
one single transmitter element (27) of the multi-threshold sensor unit (24) is arranged inside the means (21) for triggering the fixing means (20).

7. The drive device (1) according to any one of the clams 1 to 6,
**characterized in that**
one single transmitter element (27) of the multi-threshold sensor unit (24) has a pre-travel of a value between 2 mm and 10 mm, preferably of a value of 4 mm, before a first stage of the two-stage upstream detection means (28) can be triggered by the single transmitter element (27).

8. The drive device (1) according to any one of the claims 1 to 7,
**characterized in that**
the drive device (1) includes at least one joint control means (34) operating as a function of the detecting means (23) for automatic radial engagement of the at least one radially movable nut shell (12, 13) at the advancing spindle (8) and for initiating a reference travel of the main bearing means (17) to be carried out by means of the radially engaged advancing spindle nut (9) up to the infusion syringe piston (4).

9. The drive device (1) according to claim 8,
**characterized in that**
the joint control means (34) comprises a comparator circuit unit (100) for switching holding solenoids (80, 81).

10. The drive device (1) according to any one of the claims 1 to 9,
**characterized in that**
the advancing means (6) comprises holding solenoids (80, 81) controllable by means of the detecting means (23) for actuating axially movable sliding sleeves (53, 56) for radially engaging the advancing spindle nut (9) vis-à-vis the advancing spindle (8).

11. An infusion pump (2) comprising a drive head unit (3) for moving an infusion syringe piston (4) of an infusion syringe (5) disposed on the infusion pump (2) and comprising an advancing means (6) for driving the drive head unit (3),
**characterized by**
a drive device (1) according to any one of the preceding claims.

12. A method for start-up of an infusion syringe (5) on an infusion pump (2) comprising a drive head unit (3) for linear movement of an infusion syringe piston (4) in which at least one radially movable nut shell (12, 13) of an advancing spindle nut (9) is radially disengaged from an advancing spindle (8) of an advancing means (6) for driving the drive head unit (3) so that the drive head unit (3) of a piston plate (18) of the infusion syringe piston (4) can be quickly axially fed by hand and in which for preventing inadvertent bolus administration the piston plate (18) can be detected by a detecting means (23) including a multi-threshold sensor unit (24) and an at least two-stage upstream detection means (28) associated with the drive head unit (3), before the piston plate (18) enters into contact with a main bearing surface (19) of a main bearing means (17) of the drive head unit (3), wherein
- in a first detecting step a contact element (27A) of the detecting means (23) triggers a first sensor element (29), thereby the manual feeding of the drive head unit (3) by means of the advancing spindle nut (9) and the advancing spindle (8) being blocked in that the at least one radially movable nut shell (12, 13) is radially engaged on the advancing spindle (8), wherein then the drive head unit (3) of the piston plate (18) is further fed by motor drive manner via the advancing means (6),
- in a second detecting step the contact element (27A) triggers a second sensor element (30), thereby a motor-driven reference travel of the drive head unit (3) up to the main bearing surface (19) of the main bearing unit (17) being initiated, and
- in a third detecting step the motor-driven reference travel is stopped in the course of contact of the piston plate (18) with the main bearing surface (19) and retaining brackets (20A, 20B) of a fixing means (20) for the piston plate (18) are closed, thereby the piston plate (18) being secured to the main bearing means (17).

13. The method according to claim 12,
**characterized in that**
the manual infeed of the drive head unit (3) is blocked, before the contact element (27A) triggers the second sensor element (30).

14. The method according to claim 12 or 13,
**characterized in that**
the motor-driven reference travel is carried out by way of a defined number of motor steps.

## Revendications

1. Dispositif d'entraînement (1) pour le déplacement linéaire d'un piston (4) d'une seringue à perfusion (5) disposée sur une pompe à perfusion (2), comportant une unité de tête d'entraînement (3) munie d'un dispositif de support principal (17) pour supporter le piston (4) de la seringue à perfusion, un dispositif d'immobilisation (20) pour immobiliser ledit piston (4) sur le dispositif de support principal (17), ainsi que des moyens (21) pour débloquer le dispositif d'immobilisation (20), et comportant un dispositif de poussée (6) pour l'unité de tête d'entraînement (1), muni d'une broche de poussée (8) actionnable par moteur et d'un écrou pour broche de poussée (9) en plusieurs parties, comportant au moins une coque d'écrou (12, 13) mobile radialement, ladite unité de tête d'entraînement (3) comportant en outre un dispositif de détection (23) destiné à détecter le piston (4) de la seringue à perfusion en amont du dispositif de support principal (17),
**caractérisé en ce que**
le dispositif de détection (23) comporte une unité de capteurs (24) à plusieurs valeurs seuil comportant un dispositif de détection préalable (28) à deux niveaux au moins, destiné à détecter différentes positions du piston (4) de la seringue à perfusion, avant qu'un plateau (18) du piston (4) n'entre en contact avec le dispositif de support principal (17).

2. Dispositif d'entraînement (1) selon la revendication 1, **caractérisé en ce que** l'unité de capteurs (24) à plusieurs valeurs seuil comporte au moins deux capteurs (29, 30) déblocables au moyen d'un seul élément transmetteur (27), ledit élément transmetteur (27) comportant un élément de contact (27A) s'avançant au-delà du dispositif de support principal (17).

3. Dispositif d'entraînement (1) selon la revendication 1 ou 2, **caractérisé en ce que** le dispositif de détection (23) comporte au moins deux barrières lumineuses (29, 30) qui sont disposées axialement l'une derrière l'autre à l'intérieur de l'unité de tête d'entraînement (3).

4. Dispositif d'entraînement (1) selon la revendication 3, **caractérisé en ce qu'**au moins l'une des deux barrières lumineuses (29, 30) est réalisée sous la forme d'une barrière lumineuses à fourche.

5. Dispositif d'entraînement (1) selon l'une quelconque des revendications 1 à 4, **caractérisé en ce que** le dispositif de support principal (17) comporte un plateau à membrane (22), qui comprend un élément transmetteur (27), comportant un élément de contact (27A), destiné à commander les capteurs (29, 30) de l'unité de capteurs (24) à plusieurs valeurs seuil.

6. Dispositif d'entraînement (1) selon l'une quelconque des revendications 1 à 5, **caractérisé en ce qu'**un seul élément transmetteur (27) de l'unité de capteurs (24) à plusieurs valeurs seuil est agencé à l'intérieur des moyens (21) destinés à débloquer le dispositif d'immobilisation (20).

7. Dispositif d'entraînement (1) selon l'une quelconque des revendications 1 à 6, **caractérisé en ce qu'**un seul élément transmetteur (27) de l'unité de capteurs (24) à plusieurs valeurs seuil comporte une pré-course ayant une valeur entre 2 mm et 10 mm, de préférence ayant une valeur de 4 mm, avant qu'un premier niveau du dispositif de détection préalable (28) à deux niveaux ne soit débloqué par le seul élément transmetteur (27).

8. Dispositif d'entraînement (1) selon l'une quelconque des revendications 1 à 7, **caractérisé en ce que** ledit dispositif d'entraînement (1) comporte au moins un dispositif de réglage et/ou commande (34) commun, opérant en fonction du dispositif de détection (23), pour l'engrènement radial automatique de ladite au moins une coque d'écrou (12, 13) mobile radialement sur la broche de poussée (8) et pour initier un déplacement de référence du dispositif de support principal (17), actionnable au moyen de l'écrou pour broche de poussée (9) engrené radialement, jusqu'au piston (4) de la seringue à perfusion.

9. Dispositif d'entraînement (1) selon la revendication 8, **caractérisé en ce que** le dispositif de réglage et/ou commande (34) commun comporte une unité de commande de comparaison (100) destinée à commander les électro-aimants de retenue (80, 81).

10. Dispositif d'entraînement (1) selon l'une quelconque des revendications 1 à 9, **caractérisé en ce que** le dispositif de poussée (6) comporte des électro-aimants de retenue (80, 81) propres à être commandés par le dispositif de détection (23) et destinés à actionner des fourreaux coulissants (53, 56) mobiles axialement pour l'engrènement radial de l'écrou pour broche de poussée (9) par rapport à la broche de poussée (8).

11. Pompe à perfusion (2) comportant une unité de tête d'entraînement (3) pour le déplacement d'un piston (4) d'une seringue à perfusion (5) disposée sur la pompe à perfusion (2), et comportant un dispositif de poussée (6) pour l'actionnement de l'unité de tête d'entraînement (3),
**caractérisé par**
un dispositif d'entraînement (1) selon l'une quelconque des revendications précédentes.

12. Procédé pour l'actionnement d'une seringue à perfusion (5) sur une pompe à perfusion (2), comportant une unité de tête d'entraînement (3) pour le déplacement linéaire d'un piston (4) d'une seringue à perfusion, dans lequel au moins une coque d'écrou (12, 13) mobile radialement d'un écrou pour broche de poussée (9) peut être désengrenée radialement par une broche de poussée (8) d'un dispositif de poussée (6) pour actionner l'unité de tête d'entraînement (3), de telle sorte que l'unité de tête d'entraînement (3) peut être avancée axialement rapidement à la main vers un plateau de piston (18) du piston (4) de la seringue à perfusion, et dans lequel pour empêcher un effet bolus involontaire, le plateau de piston (18) peut être détecté à cet effet par un dispositif de détection (23), associé à l'unité de tête d'entraînement (3) et muni d'une unité de capteurs (24) à plusieurs valeurs seuil et d'un dispositif de détection préalable (28) à deux niveaux au moins, avant que le plateau de piston (18) n'entre en contact avec une surface de support principale (19) d'un dispositif de support principal (17) de l'unité de tête d'entraînement (3), dans lequel procédé
- dans une première étape de détection, un élément de contact (27A) du dispositif de détection (23) débloque un premier élément capteur (29), moyennant quoi l'avance manuelle de l'unité de tête d'entraînement (3) au moyen de l'écrou pour broche de poussée (9) et de la broche de poussée (8) est bloquée, par le fait que ladite au moins une coque d'écrou (12, 13) mobile radialement est engrenée radialement sur la broche de poussée (8), l'unité de tête d'entraînement (3) continuant, ensuite, à être avancée par un moteur vers le plateau de piston (18) au moyen du dispositif de poussée (6),
- dans une deuxième étape de détection, l'élément de contact (27A) débloque un deuxième élément capteur (30), ce qui initie le déplacement de référence, actionné par un moteur, de l'unité de tête d'entraînement (3) jusqu'au niveau de la surface de support principale (19) du dispositif de support principal (17), et
- dans une troisième étape de détection, sous l'effet d'un contact du plateau de piston (18) avec la surface de support principale (19), le déplacement de référence, actionné par un moteur, est stoppé et des étriers de retenue (20A, 20B) d'un dispositif d'immobilisation (20) pour le plateau de piston (18) se ferment, moyennant quoi le plateau de piston (18) est immobilisé au niveau du dispositif de support principal (17).

13. Procédé selon la revendication 12, **caractérisé en ce que** l'avance manuelle de l'unité de tête d'entraînement (3) est bloquée avant que l'élément de contact (27A) ne débloque le deuxième élément capteur (30).

14. Procédé selon la revendication 12 ou 13, **caractérisé en ce que** le déplacement de référence, actionné par un moteur, est mis en oeuvre à l'appui d'un nombre défini de pas du moteur.
